# EUROPEAN PATENT APPLICATION

(11) **EP 2 314 720 A1**
(43) Date of publication of application: **27.04.2011**
(21) Application number: 09305995.4
(22) Date of filing: 20.10.2009
(51) Int. Cl.: C12Q 1/70

(54) **Detection of respiratory viruses**

(71) Applicant: Assistance Publique Hôpitaux De Paris, 75004 Paris 4 (FR)
(72) Inventor: The designation of the inventor has not yet been filed
(74) Representative: Gallois, Valérie

(57) **Abstract**

The present invention relates to a set of nucleic acids appropriate for simultaneously detecting respiratory viruses including FLUAV, FLUBV and RSV, kits comprising this set and methods using it.

SEQUENCE LISTING

<110>  Assistance Publique - Hopitaux de Paris

<120>  DETECTION OF RESPIRATORY VIRUSES

<130>  B957EP

<160>  9

<170>  PatentIn version 3.3

<210>  1
<211>  23
<212>  DNA
<213>  artificial sequence

<220>
<223>  primer

<400>  1
aggctctcat ggartggcta aag                                              23

<210>  2
<211>  20
<212>  DNA
<213>  artificial sequence

<220>
<223>  primer

<400>  2
acggtgagcg tgaacacaaa                                                  20

<210>  3
<211>  18
<212>  DNA
<213>  artificial sequence

<220>
<223>  probe

<400>  3
tgtcacctct gactaagg                                                    18

<210>  4
<211>  21
<212>  DNA
<213>  artificial sequence

<220>
<223>  primer

<400>  4
tgtcgctgtt tggagacaca a                                                21

<210>  5
<211>  21
<212>  DNA
<213>  artificial sequence

<220>
<223>  primer

<400>  5

tgctttgcct tctccatctt c                                                21

<210>  6
<211>  16
<212>  DNA
<213>  artificial sequence

<220>
<223>  probe

<400>  6
tgcctacctg ctttca                                                      16

<210>  7
<211>  30
<212>  DNA
<213>  artificial sequence

<220>
<223>  primer

<400>  7
gtggaacttc atcctgacat aagatatatt                                       30

<210>  8
<211>  23
<212>  DNA
<213>  artificial sequence

<220>
<223>  primer

<400>  8
gttgcatctg tagcaggaat ggt                                              23

<210>  9
<211>  23
<212>  DNA
<213>  artificial sequence

<220>
<223>  probe

<400>  9
attgcaatga tcatagttta cct                                              23

## Description

### FIELD OF THE INVENTION

The present invention relates to the field of diagnosis, in particular to the simultaneous detection of multiple respiratory viruses including influenza virus A, influenza virus B and respiratory syncytial virus.

### BACKGROUND OF THE INVENTION

The burden of viral respiratory infection is mostly represented, in the northern hemisphere, by the seasonal epidemic circulation of influenza A (FLUAV), influenza B (FLUBV) and human respiratory syncytial virus (RSV), especially in infants under 96 weeks of age and in elderly patients. Other viruses, such as human metapneumovirus (hMPV), parainfluenza viruses (PIV), adenovirus and human rhinovirus may also be involved in severe respiratory diseases, although with lower rates of circulation. Antigen detection by the means of immunofluorescence (DIF) is the usual first-line diagnostic approach, because it is simple to perform and provides rapid identification of most common agents. Although very specific, DIF antigen detection lacks sensitivity unless associated to cell-culture. Non-detection of actual viral infections is frequent among children, but even more among adult patients, who shed lower viral loads in upper respiratory tract secretions and for whom sample collection is often less reliable than in children.

An alternative to DIF antigen detection is the detection of viral genomes by the means of molecular approaches. Various in-house or commercial assays are available, each presenting with advantages and drawbacks. However, to date, first-line molecular approach is not routinely performed, either because of high cost (commercial assays) or because only few in-house assays are multiplex and have short turnaround times (Gunson RN, J Clin Virol 2005, 33:341-4; Templeton KE, J Clin Microbiol 2004, 42:1564-9; Boivin G, J Clin Microbiol 2004, 42:45-51; Bellau-Pujol S, J Virol Meth 2005, 126:53-63; Bonroy C, Clin Microbiol Infect 2007, 13:504-9; Brittain-Long R, J Clin Virol 2008, 41:53-6). Methods for detecting seven most common viruses that cause respiratory infections are described in US 6,881,835 and WO97/16570. WO2006/121773 discloses a detection assay for influenza A and B viruses. WO2009/085733 discloses a multiplex detection assay for influenza and RSV viruses.

Rapid and specific diagnosis of influenza A virus (FLUAV), influenza B (FLUBV) and respiratory syncytial virus (RSV) impacts monitoring of the patients in various aspects such as hospital admission or discharge, antibiotics administration, antiviral therapy and differential diagnosis investigation. In the inventors' experience, the detection of RSV infection in infants less than 6 weeks old, who do not present with undeniable respiratory failure requiring hospitalization, leads to hospital admission to monitor the risk of rapid respiratory aggravation or apnea. A similar attitude is applied to children less than 48 weeks with a medical condition such as prematurity, or underlying cardiopulmonary disease. Children with influenza often present with alarming neurological symptoms prevailing on respiratory disease: rapid and specific identification of influenza infection supports the clinician's decision to limit further investigation. Little is known about incidence and severity associated to co-infections by FLUAV and RSV. In adult patients, rapid identification of influenza infection may lead to oseltamivir administration and changes in antibiotic treatment; patients will be discharged as soon as possible to avoid nosocomial transmission of the virus. In a pandemic situation occurring during RSV circulation, the distinction of patients infected by FLUAV from those infected by RSV, and the detection of those infected by both agents would be of paramount value. Cost-effective strategies involving sensitive and accurate molecular approaches are needed to improve routine management of viral respiratory infections, particularly in the context of a pandemic situation.

### SUMMARY

The inventors have developed a sensitive and specific, simple one-step triplex real-time RT-PCR assay, allowing the simultaneous detection of RSV, FLUAV and FLUBV genomes from biological samples, preferably respiratory samples. This assay was performed daily during the 2008-2009 winter season in a teaching-hospital laboratory setting handling samples from patients hospitalized in pediatric or intensive care units. Rapid and accurate identification of viral infections both in children and adults turned out to be a cost-effective strategy improving the management of patients presenting with severe forms of respiratory infection. This assay allows the detection of mixed infections.

Accordingly, the present invention relates to a set of nucleic acids, useful for the simultaneous detection of influenza A virus (FLUAV), influenza B virus (FLUBV) and respiratory syncytial virus (RSV) in a biological sample, the set comprising:
- a first pair of primers and a first probe, specific of the M gene of human influenza A;
- a second pair of primers and a second probe, specific of the M gene of human influenza B; and
- a third pair of primers and a third probe, specific of the L gene of human respiratory syncytial virus.

In a first embodiment, the third pair of primers has the sequences of SEQ ID Nos 7 and 8 and preferably the third probe has the sequence of SEQ ID No 9 or a complement thereof.

In a second embodiment, the first pair of primers has the sequences of SEQ ID Nos 1 and 2 and preferably the first probe has the sequence of SEQ ID No 3 or a complement thereof.

In a third embodiment, the second pair of primers has the sequences of SEQ ID Nos 4 and 5 and preferably the second probe has the sequence of SEQ ID No 6 or a complement thereof.

In a more preferred embodiment, said first pair of primers has the sequences of SEQ ID Nos 1 and 2 and said first probe has the sequence of SEQ ID No 3 or a complement thereof; said second pair of primers has the sequences of SEQ ID Nos 4 and 5 and said second probe has the sequence of SEQ ID No 6 or a complement thereof; and said third pair of primers has the sequences of SEQ ID Nos 7 and 8 and said third probe has the sequence of SEQ ID No 9 or a complement thereof.

Optionally, the set according to the present invention further comprises one or several set(s) of pair of primers and probe for detecting other respiratory viruses such as human metapneumovirus (hMPV), parainfluenza viruses (PIV), adenovirus and human rhinovirus.

The present invention also relates to a method for the simultaneous detection of influenza A virus (FLUAV), influenza B virus (FLUBV) and respiratory syncytial virus (RSV) in a biological sample from a patient, comprising :
- providing a biological sample from a patient;
- extracting viral RNA from the biological sample;
- carrying out a RT-PCR with the set of nucleic acids according to the present invention; and
- detecting amplification products for each virus among FLUAV, FLUBV and RSV, thereby the presence of an amplification product is indicative of the presence of the virus in the biological sample.

In an alternative method, instead of carrying a RT-PCR, the method comprises a step of reverse-transcription and a step of PCR amplication.

The present invention further concerns the use of a set of nucleic acids according to the present invention for simultaneously detecting respiratory viruses including RSV, FLUAV and FLUBV. It further concerns a method of simultaneously detecting respiratory viruses including RSV, FLUAV and FLUBV by using a set of nucleic acids according to the present invention. In addition, it concerns a set of nucleic acids according to the present invention for preparing a diagnostic kit useful for simultaneously detecting respiratory viruses including RSV, FLUAV and FLUBV. Optionally, the kit further comprises other components such as a DNA polymerase, a reverse-transcriptase, RNase inhibitors, dNTPs or a PCR buffer.

### BRIEF DESCRIPTION OF THE DRA WINGS

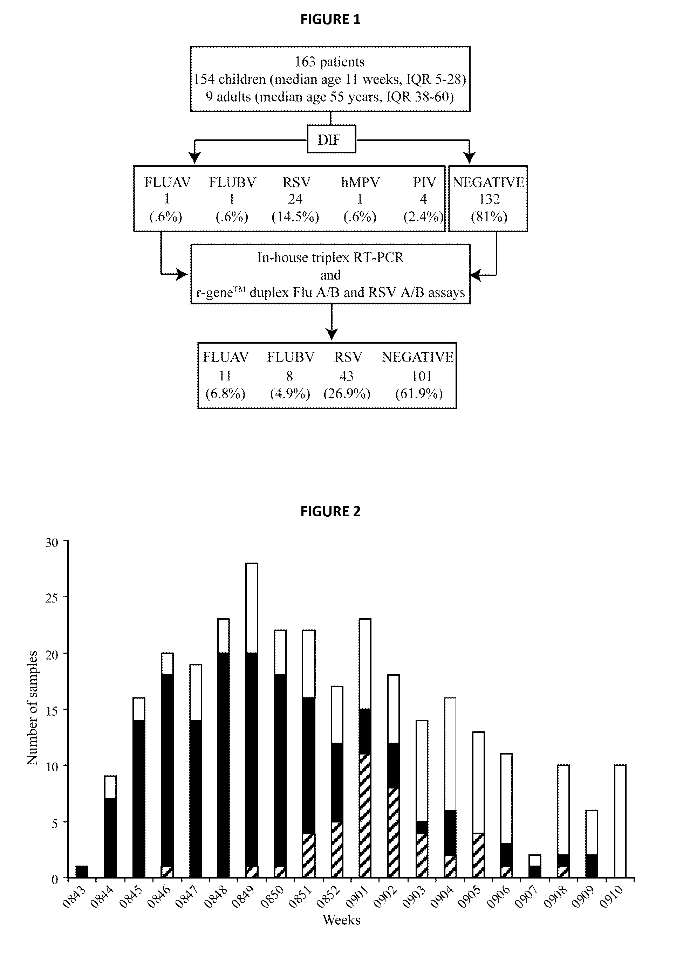

### Figure 1 : Ability of the triplex RT-PCR assay of the present invention to detect viral infections from nasopharyngeal samples

163 samples routinely tested by DIF were re-tested by the use of the assay of the invention and the commercial Flu A/B and RSV A/B r-gene™ assays. Samples found positive for FLUAV, FLUBV or RSV by DIF were also positive with both molecular tests. Of 132 samples found negative by DIF, 41 (31%) were found positive by RT-PCR, with 100% concordance between both in-house and commercial assays, although C_{T} values were lower with the r-gene™ tests than with the triplex assay (FLUAV, *P*=.0077, FLUBV, *P*=.0180, RSVA, *P*=.0280, RSVB, *P*=.0380, by the use of the Wilcoxon rank-sum test).

### Figure 2 :Temporal distribution of the samples

The number of samples positive for RSV (black boxes) or FLUAV (dashed boxes), or negative (white boxes) is reported for each week.

### Figure 3 : Age distribution of the RSV infection as compared to FLUAV and negative among children aged <120 weeks (n=252)

Children with RSV infection were significantly younger than those with FLUAV or those found negative (Wilcoxon. rank-sum test). Bars indicate range of ages (minimum-maximum). Boxes represent the number of cases included in the IQR, with median age represented by plain line in the box.

### DETAILED DESCRIPTION

The aim of the inventors was to improve diagnosis efficiency of severe cases of infection by respiratory viruses in the context of high attack rates of infections including the concomitant circulation of three major agents eventually involved in mixed infections. The higher sensitivity of RT-PCR as compared to DIF prompted them to propose an alternative diagnosis strategy, combining the use of a molecular approach targeting FLUAV, FLUBV and RSV, during their specific epidemic circulation. To be efficient, this strategy should allow providing a prompt result whatever the approach (DIF or RT-PCR of the present invention) by processing samples once daily.

To achieve this purpose, the inventors developed an inexpensive, rapid (less than 4h), specific, sensitive and one-step triplex real-time RT-PCR assay to detect FLUAV, FLUBV and RSV (in particular RSV-A and RSV-B) from biological samples, preferably respiratory samples. Specificity, sensitivity and efficiency of the assay were evaluated on cell-culture supernatants and RNA extracts. The assay was compared to antigen detection by direct immunofluorescence (DIF) and to the commercial RT-PCR duplex Flu A/B and RSV A/B r-gene™ assays. Limit of detection from culture supernatant was 1-10 pfu/input (FLUAV and FLUBV), and 2x10⁻² TCID₅₀/input (RSV). Quantified *in vitro* transcribed FLUAV RNA was detected at 10³ copies/input. RT-PCR efficiencies were similar for all viruses. Ability to detect viral genomes from respiratory samples was identical to that of the r-gene™ assay. The assay according to the present invention was able to detect viral genomes corresponding to FLUAV (seasonal H1N1 and H3N2, novel 2009-H1N1), FLUBV, RSV-A and RSV-B from respiratory samples, and was routinely used for diagnosis from November 2008 to March 2009, resulting in 63% positive results from 298 samples (145 positive for RSV and 42 for FLUAV). In the perspective of a pandemic situation, an inexpensive and user-friendly molecular tool that distinguishes FLUAV (including the novel 2009-H1N1 variant) from FLUBV and RSV, and detects mixed infections, should present utmost usefulness. Indeed, one of the main issues of this triplex real-time RT-PCR was to overcome PCR competition, especially between FLUBV and RSV (because of the difference between amplicon sizes - see table 2).

In conclusion, the inventors provide an alternate approach to improve the routine detection of respiratory virus infections in the context of high rates of infection. This strategy combines the use of a user-friendly triplex RT-PCR targeting influenza viruses and RSV during epidemic season and optionally DIF targeting additional common viruses outside influenza and RSV epidemics. In the present context of influenza A (H1N1) pandemics, the availability of a molecular tool allowing to rapidly distinguishing FLUAV from FLUBV or RSV in a general hospital setting is worth mentioning.

Accordingly, the present invention relates to a set of nucleic acids, useful for the simultaneous detection of FLUAV, FLUBV and RSV in a biological sample, the set comprising:
a) a first pair of primers and a first probe specific of the M gene of human influenza A;
b) a second pair of primers and a second probe specific of the M gene of human influenza B; and
c) a third pair of primers and a third probe specific of the L gene of human respiratory syncytial virus.

Preferably, each primer has 14-40 (e.g., 15-35, 15-25 or 15-20) nucleotides in length. Preferably, each probe has 14-100 (e.g., 16-50 or 16-25) nucleotides in length. The set of primers pair and probe is specific of the most conserved sequences of the gene for each virus.

Preferably, said first pair of primers has the sequences of SEQ ID Nos 1 and 2 and said first probe has the sequence of SEQ ID No 3. Preferably, said second pair of primers has the sequences of SEQ ID Nos 4 and 5 and said second probe has the sequence of SEQ ID No 6. Preferably, said third pair of primers has the sequences of SEQ ID Nos 7 and 8 and said third probe has the sequence of SEQ ID No 9. More preferably, said first pair of primers has the sequences of SEQ ID Nos 1 and 2 and said first probe has the sequence of SEQ ID No 3; said second pair of primers has the sequences of SEQ ID Nos 4 and 5 and said second probe has the sequence of SEQ ID No 6; and said third pair of primers has the sequences of SEQ ID Nos 7 and 8 and said third probe has the sequence of SEQ ID No 9. Preferably, the primers and probes are prepared as a mixture thereof.

The set of nucleic acids may further comprise other sets of pair of primers and probe specific for respiratory viruses such as human metapneumovirus (hMPV), parainfluenza viruses (PIV), adenovirus and human rhinovirus. In this embodiment, the pair of primers and probes for detecting hMPV are preferably specific of the M2 gene of hMPV.

The multiplex real-time RT-PCR assay preferably uses the TaqMan® PCR detection system. Information about the technology is available for instance in "QuantiTect® Multiplex PCR Handbook (www1.qiagen.com/HB/QuantitectMultiplexPCR) (the disclosure of which being incorporated herein by reference). Each probe comprises a fluorescent dye and a quencher. Preferably, the fluorescent dye is located at the 5' end of the probe and the quencher at its 3' end so that the quencher is able to take up the fluorescence of the dye. Non-exhaustive examples of fluorescent dyes are the followings: FAM, TET, JOE, VIC, Yakima Yellow, HEX, Bodipy® TMR, NED, Cy3, TAMRA, Cy3.5, ROX, Texas Red and Cy5. The quencher for the probes can be the same or different. Preferably, the quencher is non-fluorescent. Non-exhaustive examples of non-fluorescent quenchers are the followings: BHQ® (Biosearch Technology, Inc) and MGB (Minor Groove Binder). Each probe comprises a different fluorescent dye (i.e., the first probe has a first fluorescent dye, the second probe has a second dye and the third probe has a third dye, the three fluorescent dyes are chosen so as to be conveniently discriminated). The different fluorescent dyes are preferably chosen so as to have similar or near or overlapping excitation wavelengths but different or separate emission wavelengths (with a minimal overlap). In a preferred embodiment, the three fluorescent dyes are FAM, VIC and NED and the quencher is MGB. In a particular embodiment, the first probe is attached to VIC, the second probe is attached to NED, the third probe is attached to FAM and the three probes are attached to the MGB quencher (See Table 2). Obviously, the one skilled in the art can also select another convenient combination of three dyes, for instance FAM, HEX and Bodipy® TMR; FAM, HEX and NED; FAM, JOE and Bodipy® TMR; FAM, JOE and NED; FAM, VIC and Bodipy® TMR; etc... During the extension phase of PCR, the probes are cleaved by the 5'→3' exonuclease activity of Taq DNA polymerase, separating the fluorescent dye and the quencher. This results in detectable fluorescence that is proportional to the amount of accumulated PCR product.

Preferably, the biological sample is provided from a patient having symptoms of respiratory infection. However, a biological sample providing from a patient without such symptoms is also contemplated by the present invention as a preventive measure. The biological sample may be a tissue, a biological fluid or microorganisms collected from the patient, preferably a respiratory sample. Biological sample can be for instance mucus, sputum, bronchial alveolar lavage, bronchial wash, blood, urine, plasma, or serum. More preferably, the sample is nasopharyngeal aspirates, swabs or washes.

The methods for the viral RNA extraction from the sample are well-known by the one skilled in the art (See Maniatis et al., Molecular Cloning, A Laboratory Manual, 2d, Cold Spring Harbor Laboratory Press, page 16.54 (1989)) and commercially available kits exist (QIAamp™ mini blood kit, QIAamp™ Mini Viral RNA kit, Agencourt Genfind™, Roche Cobas® Roche MagNA Pure® or phenol xhloro form extraction using Eppendorf Phase Lock Gels®).

The amplification products may be detected by any method known by the one skilled in the art. For instance, the amplification products can be detected by gel electrophoresis and staining or any other appropriate method. However, the method of the invention preferably uses the most efficient way to detect the amplification products, namely a multiplex real time RT-PCR. The extracted RNA is added in a reaction mixture including the set of nucleic acids of the present invention and the appropriate reagents for the RT-PCR. Those reagents include, without being exhaustive, a reverse-transcriptase, a DNA polymerase (e.g., Taq polymerase), RNase inhibitors, dNTPs and convenient PCR buffers. Such reagents may be provided as a commercial kit, for instance the TaqMan® One-Step RT-PCR Master Mix Reagents Kit (Applera, Courtaboeuf, France) or any equivalent thereof. The first step of it is a retro-transcription, followed by a denaturing step and then the amplification cycles. The reactions, data acquisition and analyses can be carried out with any convenient cycler, for instance with the ABI PRISM 7900 HT Fast Real-Time PCR System (Applera, Courtaboeuf, France). In a preferred embodiment, the real-time RT-PCR may comprise a retro-transcription step of 30 min at 48°C, a denaturing step of 10 min at 95°C and 45 cycles of amplification including 15 sec at 95°C and 90 sec at 59°C. Optionally, each run includes a negative control (e.g., PBS) and/or a positive control of one or each target RNA (i.e., FLUAV, FLUBV and RSV). In a preferred positive control, the target RNA for each virus simultaneously detected is included as a mixture (e.g., the FLUAV, FLUBV and RSV RNA or part thereof comprising the targeted gene). Alternatively, a positive control can be included separately for each virus to be detected. The amplification products detection is carried out through the measurement of fluorescence for each dye. Accordingly, the fluorescence detection for one specific dye is indicative of the presence of the viral RNA corresponding to the probe attached to this dye, and therefore to the presence of the virus in the sample.

Of course, the present invention also contemplates an alternative method in which the RNA extract is previously reverse-transcripted into cDNA and then, instead of a RT-PCR, the method includes a PCR.

The present invention further concerns the use of a set of nucleic acids according to the present invention for simultaneously detecting respiratory viruses including RSV, FLUAV and FLUBV, preferably by performing a one-step real-time RT-PCR assay. It further concerns a method of simultaneously detecting respiratory viruses including RSV, FLUAV and FLUBV by using a set of nucleic acids according to the present invention, in particular for carrying out a one-step real-time multiplex RT-PCR. In addition, it concerns a set of nucleic acids according to the present invention for preparing a diagnostic kit useful for simultaneously detecting respiratory viruses including RSV, FLUAV and FLUBV. Optionally, the kit further comprises other components such as a DNA polymerase, a PCR buffer or a solid support on which one or more specific probes are immobilized.

The present invention is further illustrated by the following examples, which should not be construed as limiting in any way.

### EXAMPLES

### RESULTS

### Specificity, sensitivity and efficiency of the one-step real-time triplex RT-PCR assay

Specificity of the one-step real-time triplex RT-PCR assay was ascertained by the negative RT-PCR results obtained after 45 cycles of amplification of viral genome extracted from culture supernatants of negative control viruses. No cross-amplification was observed in the triplex assay between FLUAV, FLUBV and RSV genomes. The triplex positive control gave repeatedly positive signals for each corresponding dye. Sensitivity of the assay (reported in table 1) was evaluated from culture supernatants of known titers (FLUAV, FLUBV and RSV-A), and from quantified *in vitro* transcribed RNA (FLUAV). To determine RT-PCR efficiency, the inventors tested 4 tenfold serial dilutions of each RNA extract obtained from culture supernatants; efficiency was deduced from the slope of the log-linear phase as [10(-^{1/slope})-1] x 100 (table 1). To assess the ability of the assay to detect the novel influenza A (H1N1) virus, the inventors compared primers and probe sequences with all the segment 7 novel influenza A (H1N1) sequences available at www.ncbi.nlm.nih.gov/genomes/FLU/SwineFlu.html: primers and probe sequences were 100% concordant with all available sequences. Culture supernatant obtained from a clinical isolate characterized as novel influenza A (H1N1) was tested and found positive (C_{T}: 33.81).

Sensitivity and specificity were further assessed on 163 clinical samples routinely tested by DIF during the 2007-2008 winter season and kept frozen at -80°C. RNA extracts were tested both by the triplex RT-PCR assay according to the present invention and the RT-PCR Flu and RSV A/B r-gene^{™} assay. Extraction step and PCR inhibitor detection were monitored by the DICO Extra r- gene^{™} control. Results are presented figure 1 and Table 3. DIF positive samples for FLUAV, FLUBV and RSV were all found positive with both molecular tests. Of the 132 DIF negative samples, 36 were found positive by both assay according to the present invention and r-gene^{™} assays with 100% concordance between the two tests. This result indicated that sensitivity of detection of the triplex assay of the present invention was similar to that of the commercial duplex Flu A/B and RSV A/B assays, although median C_{T} values were significantly lower with the r-gene™ tests than with the triplex assay (figure 1).

### Routine monitoring of viral respiratory infections during the 2008-2009 epidemic season by the use of the one-step real-time triplex RT-PCR assay

From November 2008 to March 2009 (weeks 0843-0911), 298 patients (257 children either admitted to the pediatric unit or consulting the emergency ward and 41 adults hospitalized in the intensive-care unit for severe respiratory or influenza-like illness) were routinely sampled for genome detection of FLUAV, FLUBV and RSV, by the means of the triplex assay according to the present invention. The median number of samples was 16 per week (IQR 10-21), mostly between weeks 0845-0906 (figure 2). RT-PCR was positive in 188 (63.1%) patients: 145 (77.5%) for RSV, 42 (22.3%) for FLUAV and one for both RSV and FLUAV. No FLUBV infection was detected during this period. Median normalized C_{T} values were 30.35 (ranging from 20.04 to 42.15) for RSV and 29.31 (ranging from 20.45 to 37.12) for FLUAV. Temporal distribution of virus detection, represented on figure 2, indicated that RSV was mostly prevalent between weeks 0844-0852, and FLUAV between weeks 0851-0905. In adult patients, FLUAV represented 77.3% of viral infections, while in children; RSV represented 83% of the infections.

Cell-culture adaptation and consecutive typing of FLUAV samples was obtained for 23 (56%) samples, which all corresponded to the current epidemic strain A/Brisbane/10/07 (H3N2). Negative cell-culture results were significantly related to low positive PCR results, as indicated by with higher C_{T} levels (*P*<.0001, by the use of the Mann-Whitney U-test).

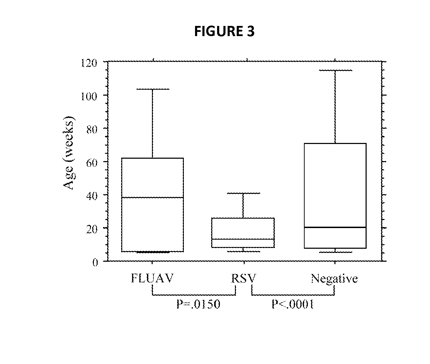

Major clinical features of the pediatric patients are reported in table 4. The inventors observed that children with RSV infection were significantly younger than those with FLUAV (*P*=.0068) or with negative RT-PCR (*P*<.0001) (figure 3). Predictive factors of evolution towards a severe respiratory condition (ie: dyspnea, need for oxygen therapy, inability to eat) and longer duration of hospitalization were associated to RSV infection. Conversely, fever and neurological symptoms were associated to influenza. The inventors detected one co-infection by FLUAV and RSV in an 8 weeks-old boy who presented with a severe respiratory disease. C_{T} values were low, indicating high viral loads for both viruses (24.76 and 25.41 for FLUAV and RSV, respectively) and FLUAV was isolated in cell culture and identified as the current epidemic strain.

Clinical features of the adult patients with FLUAV or negative infection are reported in table 5. X-ray with bilateral alveolar opacities and higher C-reactive protein levels (CRP) were significantly associated to FLUAV infection (*P*=.0002 and .01, respectively).

Concordant results obtained from 62 samples found positive by the means of a commercial RT-PCR approach allowed us to validate the assay for routine detection of virus infection. The efficacy of the triplex assay was finally assessed by the routine daily practice (2008-2009 epidemic season), during which 63% of the samples were found positive. During that season, the inventors found only one mixed infection (FLUAV / RSV), and they detected no influenza B infection. However, FLUBV virus circulation rate was sporadic in France (<5%), as confirmed by the influenza-monitoring network GROG (Groupes Regionaux d'Observation de la Grippe - Regional Influenza Surveillance Group - www.grog.org). Overall, the triplex RT-PCR assay according to the present invention was efficient to detect, from nasopharyngeal samples, current epidemic strain genomes corresponding to FLUAV (including seasonal H1N1, predominant in 2007-2008, seasonal H3N2, as sub-typed by cell-culture isolation during the 2008-2009 season and novel influenza A (H1N1) variant), FLUBV, RSV-A and RSV-B. Finally, the ability of the test to detect mixed infections might be useful to evaluate their incidence and their role in severe forms of respiratory diseases.

### MATERIALS AND METHODS

### Viruses

Positive controls: Positive control strains are described in table 1. Viruses were propagated on cell culture (MDCK for FLUAV and FLUBV, MRC5 for RSV) and virus titers were determined by cell-plaque assays (plaque forming units per mL (pfu/mL) for FLUAV and FLUBV or 50% tissue culture infectious dose (TCID₅₀) for RSV).

*In vitro*-transcribed RNA of the M gene open reading frame (from the ATG to nucleotide 982) derived from H1N1 strain A/Paris/650/2006 was also used as a control.

Negative control viruses: Culture supernatants of parainfluenza viruses -1, -2, -3 and -4, human metapneumovirus B, adenovirus type 1, echovirus type 30, rhinovirus type 31, and coronavirus OC43 and 229E were used as negative control viruses.

### Clinical samples

From January-April 2008, 163 patients hospitalized or consulting the emergency ward for severe respiratory symptoms or influenza-like syndrome were routinely tested for the detection of viral agents by DIF. After analysis, all samples (nasopharyngeal aspirates or swabs) were stored at -80°C. These samples were retrospectively tested with the RT-PCR according to the present invention and the r-gene™ assay.

From November 2008 to March 2009, 298 patients were routinely tested for the detection of viral agents by the use of the RT-PCR assay according to the present invention. Clinical characteristics of the patients were collected as part of usual medical file. No additional samples than those used for diagnosis purpose were collected for the study, according to ethical standard guidelines.

### One-step real-time triplex RT-PCR for FLUAV, FLUBV and RSVA/B genome detection in respiratory samples

The assay was designed to simultaneously detect FLUAV (H1N1 or H3N2), FLUBV and RSV (A or B) genomes in nasopharyngeal aspirates or swab samples. Viral RNA was extracted from 140µL of fresh or thawed sample by the use of QIAamp Viral RNA Mini Kit (Qiagen, Courtaboeuf, France). Extract (7.5µL) was used in a 25µL RT-PCR mixture containing 4.5µL of the primers and probe mixture (table 2), and the TaqMan® One-Step RT-PCR Master Mix Reagents Kit (Applera, Courtaboeuf, France). Each run included a negative control (consisting of previously extracted PBS) and a positive control containing 2.5µL of each target RNA (FLUAV, FLUBV and RSV). The reaction consisted of a retro-transcription step of 30 min at 48°C, followed by a 10 min denaturing step at 95°C, and then 45 cycles of amplification including 15 s at 95°C and 90 s at 59°C. The reactions, data acquisition, and analyses were performed with the ABI PRISM 7900HT Fast Real-Time PCR System (Applera, Courtaboeuf, France). Cycle threshold (C_{T}) values were normalized for all assays to allow statistical comparisons: baseline fluorescence level was calculated from cycles 5-15, fluorescence threshold was placed at .07 delta Rn.

### Other virological methods

DIF antigen detection: viral antigen detection was performed on freshly sampled nasopharyngeal aspirates or swab samples by the use of monoclonal antibodies anti-influenza A Group FITC, anti-influenza B Group FITC, anti-RSV Group FITC, anti-human metapneumovirus FITC, anti-parainfluenza Group FITC and anti-adenovirus Group FITC (Argene S.A., Verniolle, France), following the manufacturer's recommendations.

Duplex real-time RT-PCR assays: viral genomes were detected by the use of the optimized primers and probes Flu A/B r-gene™ and RSV A/B r-gene™ (Argene S.A.). Viral RNA extract (10µL, procedure as described above) was used in 3 RT-PCR mixtures containing 14µL of optimized primers and probes and 1µL of OneStep RT-PCR mix (Qiagen). Extraction step and presence of PCR inhibitors were monitored by the use of DICO Extra r-gene™ (Argene S.A.) extraction and inhibition control: 7µL of the internal control IC2 was added to the sample (including the negative control) prior to extraction. An additional RT-PCR mixture containing 10µL extract, 15µL of the DICO Extra r-gene premix (DP2) and 0.17µL of the OneStep RT-PCR mix (Qiagen) was prepared. Real-time RT-PCR was carried out on a 7900HT Fast Real-Time PCR System (Applera), following cycling conditions recommended by Argene. For all assays, baseline fluorescence level was calculated from cycles 5-15, and fluorescence threshold was placed at 2.5 delta Rn.

Cell-culture identification of FLUAV viruses: Samples were inoculated onto MDCK cells. Influenza viruses were detected by hemagglutination assay with guinea-pig erythrocytes; isolate subtype was further determined by hemagglutination-inhibition, as previously described (Kendal AP, Am J Epidemiol 1979, 110:449-61).

### Statistical analysis

The nonparametric Mann-Whitney U-test and Wilcoxon rank-sum test (StatView 5.0.0.0), and the *Chi-2* test were used for statistical comparisons. Differences were considered significant at *P* values less than .05.

**Table 1: Viral strains used as positive controls**

| | Viral strains | Titers | Limit of detection*^{a}* | RT-PCR efficiency |
|---|---|---|---|---|
| | A/Brisbane/10/07(H3N2) | 1.5x10⁸ pfu/mL | 10 (38.10) | |
| | A/Wisconsin/67/05(H3N2) | 3.7x10⁸ pfu/mL | 10 (38.80) | |
| | A/California/7/04(H3N2) | 2.5x10⁸ pfu/mL | 1 (40.06) | |
| FLUAV | A/Solomon/03/06(H1N1) | 2.2x10⁶ pfu/mL | 1 (38.69) | 88% |
| | A/NewCal/20/99(H1N1) | 3.5x10⁸ pfu/mL | 10 (39.55) | |
| | A/Paris/650/06(H1N1) ^{b} | 10⁹ copies/µL | 10³ (40.80) ^{c} | |
| | B/Florida/4/06 | 2.0x10⁶ pfu/mL | 1 (41.38) | |
| FLUBV | B/Jiangsu/10/03 | 1.2x10⁷ pfu/mL | 10 (39.28) | 92% |
| | B/Malaysia/2506/04 | 1.5x10⁸ pfu/mL | 1 (42.78) | |
| RSV-A *^{d}* | ATCC VR-26 A2 Long | 10⁴ TCID₅₀/mL | 2 x 10⁻² (43.23) | |
| | Gal /Caen/CHE/86 | - | - | |
| | Ga2/Caen/BOU/00 | - | - | 85% |
| | Ga3/Caen/TIS/97 | - | - | |
| | Ga4/Caen/CAS/01 | - | - | |
| | Ga5/Caen/PAT/93 | - | - | |
| RSV-B*^{e}* | ATCC strain B1 | - | - | |
| | Gb1/Caen/WIS/03 | - | - | |
| | Gb2/Caen/VER/02 | - | - | |
| | Gb3/Caen/GAU/02 | - | - | 88% |
| | Gb4/Caen/MAR/01 | - | - | |
| | Gb5/Caen/LEN/99 | - | - | |
| | Gb6/Caen/GOD/01 | - | - | |
| ^{a} *pfu* or TCID₅₀ / input (normalized C_{T} value) | | | | |
| *^{b}* in vitro transcribed RNA containing the M gene ORF | | | | |
| *^{c}* number of RNA copies / input (normalized C_{T} value) | | | | |
| *^{d}* culture supernatant obtained from a clinical isolate of the novel influenza A (H1N1) | | | | |
| *^{e}* RSV genotypes were determined from clinical isolates by phylogenetic analysis of the G gene as in Zambon MC, Lancet 2001, 358:1410-6 | | | | |

**Table 2: Primers and probes for the one-step real-time triplex RT-PCR**

| Target | Amplicon size (bp) | Primers and probes ^{a} | Nucleotide sequence (5' to 3') | Final concentration |
|---|---|---|---|---|
| | | F | AGGCTCTCATGGAA/GTGGCTAAAG | 300 nM |
| FLUAV *^{b}* | 85 | R | ACGGTGAGCGTGAACACAAA | 300 nM |
| | | Probe | VIC-TGTCACCTCTGACTAAGG-MGB | 100 nM |
| | | F | TGTCGCTGTTTGGAGACACAA | 100 nM |
| FLUBV *^{b}* | 65 | R | TGCTTTGCCTTCTCCATCTTC | 100 nM |
| | | Probe | NED-TGCCTACCTGCTTTCA-MGB | 200 nM |
| | | F | GTGGAACTTCATCCTGACATAAGATATATT | 900 nM |
| RSV *^{b}* | 149 | R | GTTGCATCTGTAGCAGGAATGGT | 900 nM |
| | | Probe | FAM-ATTGCAATGATCATAGTTTACCT-MGB | 200 nM |
| *^{a}* F: forward primer, R: reverse primer | | | | |
| ^{b} Genome targets: FLUAV and FLUBV: segment 7 (modified from Kuypers J, J Clin Microbiol 2006, 44:2382-8; van de Pol AC, J Clin Microbiol 2007, 45:2260-2); RSV: L gene of RSV A or B | | | | |

**Table 3: Ability of the triplex RT-PCR assay of the present invention to detect viral infections from nasopharyngeal samples**

| Test | Result (n=163 samples) | | | | | |
|---|---|---|---|---|---|---|
| | FLUAV | FLUBV | RSV | hMPV | PIV | Negative |
| DIF | 1 | 1 | 24 | 1 | 4 | 132 |
| | (.6%) | (.6%) | (14.7%) | (.6%) | (2.5%) | (81%) |
| RT-PCR ^{a} | 11 | 8 | 43 | | | 91 |
| | (6.8%) | (4.9%) | (26.4%) | NT | NT | (55.8%) |
| *^{a}All* samples were also tested with the commercial Flu A/B and RSV A/B r-gene™ assays. Results were 100% concordant with the assay of the present invention, although C_{T} values were lower with the r-gene™ tests than with the triplex assay: (FLUAV, *P*=.0077, FLUBV, *P*=.0180, RSVA, *P*=.0280, RSVB, *P*=.0380, by the use of the Wilcoxon rank-sum test) | | | | | | |

**Table 4: Clinical characteristics of the pediatric patients (patient with mixed infection FLUAV/RSV was excluded)**

| Characteristics number of cases (%) | Total | RSV | FLUAV | Neg | P | | |
|---|---|---|---|---|---|---|---|
| | | | | | RSV vs Neg | FLUAV vs Neg | RSV vs FLUAV |
| Total number | 256 | 140 (54.7) | 25 (9.8) | 91 (35.5) | | | |
| Sex ratio | 1.25 | 1.1 | 2.25 | 1.33 | NS | NS | NS |
| Age in weeks*^{a}* | 13 (6-36) | 11 (6-24) | 37 (6-60) | 18 (6-73) | <.0001 | NS | .0068 |
| Fever >38°C | 98 (52.7) | 36(40.5) | 23 (95.8) | 39 (53.4) | NS | .0002 | <.0001 |
| Dyspnea | 199 (77.7) | 136 (97.1) | 4 (15.4) | 60 (66.7) | <.0001 | <.0001 | <.0001 |
| Need for oxygen therapy | 103 (40.2) | 83 (59.3) | 2 (7.7) | 17 (18.7) | <.0001 | NS | <.0001 |
| Inability to eat | 104 (40.6) | 87 (62.1) | 0 | 16 (17.6) | <.0001 | NS | <.0001 |
| Apnea | 6 (2.3) | 4 (2.9) | 0 | 2 (2.2) | NS | NS | NS |
| Abnormal chest X-ray | 61 (26.8) | 37 (28.5) | 1 (4.3) | 21 (27.6) | NS | NS | .0390 |
| Need for intensive care | 8 (3.1) | 7 (5.0) | 0 | 1 (1.1) | NS | NS | NS |
| Neurological symptom | 25 (9.8) | 0 | 12 (46.1) | 13 (14.3) | <.0001 | .0008 | <.0001 |
| Underlying condition*^{b}* | 6 (2.3) | 1 (0.7) | 0 | 5 (5.5) | .0304 | NS | NS |
| Influenza vaccination | 3 (1.2) | 1 (0.7) | 0 | 2 (2.2) | NS | NS | NS |
| Antibiotic administration | 61 (23.8) | 36 (25.7) | 4 (15.4) | 21 (23.1) | NS | NS | NS |
| Number of days of hospitalization*^{a}* | 3 (2-5) | 4 (3-6) | 2 (1-4) | 3 (2-4) | <.0001 | NS | NS |
| *^{a}* Median (IQR) | | | | | | | |
| *^{b}* Drepanocytosis, n=2; Prematurity, n=1; Abnormal deglutition, n=1; Cystic fibrosis, n=1; Splenectomy, n=1 | | | | | | | |

**Table 5: Clinical characteristics of the adult patients (patients infected by RSV were excluded)**

| Characteristics number of cases (%) | Total | FLUAV | Neg | *P* (FLUAV vs Neg) |
|---|---|---|---|---|
| Total number | 41 | 17 (41.5) | 19 (46.3) | |
| Sex ratio | .95 | .89 | 1.1 | NS |
| Median age expressed in years (IQR) | 61 (51-73) | 57 (51-73) | 59 (40-76) | .64 |
| Underlying condition*^{a}* | 27 (65.9) | 11 (64.7) | 12 (63.2) | .9 |
| Need for mechanical ventilation | 19 (46.3) | 6 (35.3) | 9 (47.4) | .38 |
| Documented bacterial infection | 10 (30.3) | 4 (36.4) | 4 (23.5) | .46 |
| Antibiotic administration | 31 (75.6) | 14 (82.4) | 12 (63.2) | .29 |
| X-ray with bilateral alveolar opacities | 13 (34.2) | 9 (56.3) | 1 (5.6) | .0002 |
| CRP levels at admission (mg/mL) | 94 (39-171) | 104(41-179) | 64 (30-155) | .01 |
| Oseltamivir administration | 6 (14.6) | 5 (29.4) | 1 (5.3) | .052 |
| Number of days of hospitalization | 2 (2-8) | 3 (1-4) | 2 (1-4) | 1 |
| Death during hospital stay | 10 (24.4) | 3 (17.6) | 5 (26.3) | .48 |
| *^{a}* namely COPD: n=11, chronic respiratory insufficiency: n=7, asthma: n=6, cardiac insufficiency: n=7 | | | | |

## Claims

1. A set of nucleic acids, useful for the simultaneous detection of influenza A virus (FLUAV), influenza B virus (FLUBV) and respiratory syncytial virus (RSV) in a biological sample, the set comprising:
a) a first pair of primers and a first probe, specific of the M gene of human influenza A;
b) a second pair of primers and a second probe, specific of the M gene of human influenza B; and
c) a third pair of primers and a third probe, specific of the L gene of human respiratory syncytial virus.

2. The set according to claim 1, wherein the third pair of primers has the sequences of SEQ ID Nos 7 and 8.

3. The set according to claim 2, wherein the third probe has the sequence of SEQ ID No 9 or a complement thereof.

4. The set according to anyone of claims 1-3, wherein the first pair of primers has the sequences of SEQ ID Nos 1 and 2.

5. The set according to claim 4, wherein the first probe has the sequence of SEQ ID No 3 or a complement thereof.

6. The set according to anyone of claims 1-5, wherein the second pair of primers has the sequences of SEQ ID Nos 4 and 5.

7. The set according to claim 6, wherein the second probe has the sequence of SEQ ID No 6 or a complement thereof.

8. The set according to claim 1, wherein said first pair of primers has the sequences of SEQ ID Nos 1 and 2 and said first probe has the sequence of SEQ ID No 3 or a complement thereof; said second pair of primers has the sequences of SEQ ID Nos 4 and 5 and said second probe has the sequence of SEQ ID No 6 or a complement thereof; and said third pair of primers has the sequences of SEQ ID Nos 7 and 8 and said third probe has the sequence of SEQ ID No 9 or a complement thereof.

9. The set according to anyone of claims, further comprising one or several set(s) of pair of primers and probe for detecting other respiratory viruses such as human metapneumovirus (hMPV), parainfluenza viruses (PIV), adenovirus and human rhinovirus.

10. A kit for simultaneously detecting respiratory viruses including RSV, FLUAV and FLUBV comprising a set of nucleic acids according to anyone of claims 1-9.

11. The kit according to claim 10, wherein the kit further comprises other components such as a DNA polymerase, a Reverse transcriptase, RNase inhibitors, or a PCR buffer.

12. A method for the simultaneous detection of influenza A virus (FLUAV), influenza B virus (FLUBV) and respiratory syncytial virus (RSV) in a biological sample from a patient, comprising :
- providing a biological sample from a patient;
- extracting viral RNA from the biological sample;
- carrying out a RT-PCR with the set of nucleic acids according anyone of claims 1-9; and
- detecting amplification products for each virus among FLUAV, FLUBV and RSV, thereby the presence of an amplification product is indicative of the presence in the biological sample of the virus.
